Europäisches Patentamt

(19) European Patent Office

Office européen des brevets

(11) EP 0 755 670 B1

(12) FASCICULE DE BREVET EUROPEEN

(45) Date de publication et mention de la délivrance du brevet:
21.05.1997 Bulletin 1997/21

(51) Int. Cl.⁶: **A61K 7/42**, A61K 7/48

(21) Numéro de dépôt: 96401304.9

(22) Date de dépôt: 14.06.1996

(54) **Compositions cosmétiques anhydres contenant des nanopigments de TiO2 et des microsphères creuses déformables**

Wasserfreie kosmetische Zusammensetzungen, welche TiO2 Nanopigmente und verformbare hohle Mikrokugeln enthalten

Anhydrous TiO2 containing nanopigments cosmetic compositions and hollow, transformable microspheres

(84) Etats contractants désignés:
AT BE CH DE ES FR GB IT LI NL SE

(30) Priorité: 13.07.1995 FR 9508563

(43) Date de publication de la demande:
29.01.1997 Bulletin 1997/05

(73) Titulaire: **L'OREAL**
75008 Paris (FR)

(72) Inventeurs:
• **Hansenne, Isabelle**
75017 Paris (FR)
• **Ribery, Delphine**
92300 Levallois-Perret (FR)
• **Guillemot, Laure**
92110 Clichy (FR)

(74) Mandataire: **Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
EP-A- 0 056 219    EP-A- 0 112 807
EP-A- 0 320 473    EP-A- 0 523 294
WO-A-92/13517      WO-A-92/21315
FR-A- 2 622 440    FR-A- 2 622 441
US-A- 3 615 972

• PATENT ABSTRACTS OF JAPAN vol. 11, no. 18 (C-398) [2465] , 17 Janvier 1987 & JP-A-61 194013 (TAISHO PHARMACEUTICAL CO. LTD)

**Description**

La présente invention concerne une nouvelle composition anhydre à usage topique plus particulièrement destinée à la photoprotection de la peau et/ou des cheveux contre le rayonnement ultraviolet ainsi que son utilisation dans le domaine cosmétique. Cette composition contient dans un support cosmétique constitué d'une phase grasse au moins un nanopigment d'oxyde de titane et des microsphères creuses déformables d'un copolymère expansé de chlorure de vinylidène et d'acrylonitrile ou de chlorure de vinylidène, d'acrylonitrile et de méthacrylate.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

Pour filtrer les rayonnements UV-A et UV-B, il existe sur le marché différents types de filtres solaires : les pigments minéraux et les filtres organiques. Ces filtres doivent pouvoir absorber ou bloquer les rayons nocifs du soleil tout en restant inoffensifs pour l'utilisateur.

Ainsi, de nombreux filtres organiques solaires capables d'absorber plus ou moins sélectivement les rayons UV nocifs ont été proposés à ce jour dans le domaine de la cosmétique. Pour diverses raisons, ces filtres ne donnent cependant pas entièrement satisfaction.

C'est pourquoi on cherche de plus en plus à éviter l'emploi de ces filtres organiques en privilégiant l'utilisation des pigments minéraux, qui jouent également le rôle de filtres solaires, principalement par diffusion / réflexion des UV, tout en apportant une sécurité plus grande pour l'utilisateur.

A ce titre, le pigment minéral le plus courant utilisé à ce jour est l'oxyde de titane de qualité nanopigmentaire, dont les propriétés filtrantes sont bien connues.

JP-A-61194013 divulgue des compositions cosmétiques contenant des pigments d'oxyde de titane et des microparticules d'un polymère.

Toutefois, il est également bien connu que le $TiO_2$, compte tenu de son caractère hydrophile, a tendance à être facilement éliminé par l'eau lors d'une baignade ou d'une douche par exemple, réduisant par à l'efficacité de la composition le contenant. Afin d'éviter cet inconvénient et d'augmenter la rémanence à l'eau du $TiO_2$, on cherche de plus en plus à préparer des produits parfaitement anhydres, en particulier des crèmes anhydres.

Par ailleurs, il est nécessaire, afin d'obtenir des facteurs de protection convenables, de pouvoir introduire dans les compositions solaires des pourcentages élevés en $TiO_2$, en particulier supérieurs à 5% en poids.

Mais, avec de telles teneurs en $TiO_2$, on observe une instabilité à la lumière des compositions contenant ces pigments dans un milieu sans oxygène, qui se manifeste par l'apparition d'une coloration bleue. Cette photocoloration, connue sous le nom de photobleuissement, n'est évidemment pas souhaitable d'un point de vue esthétique.

Un autre problème observé avec les compositions contenant des teneurs élevées en $TiO_2$ nanopigmentaire est l'apparition d'un blanchiment lorsqu'on les applique sur la peau. Ce phénomène n'est pas non plus souhaitable d'un point de vue esthétique.

Ainsi, il existe encore à ce jour un fort besoin quant à pouvoir disposer de compositions solaires anhydres contenant du $TiO_2$, qui ne présentent pas, ou du moins de manière limitée, les phénomènes de photobleuissement et de blanchiment évoqués ci-dessus.

La présente invention vise à la satisfaction d'un tel besoin.

Ainsi, à la suite d'importantes recherches menées dans le domaine de la photoprotection évoqué ci-dessus, la Demanderesse a maintenant découvert que l'introduction de microsphères creuses déformables dans une composition anhydre contenant des nanopigments de $TiO_2$ réduisait significativement les phénomènes de photobleuissement dû aux UV et de blanchiment de la composition lors de son application sur la peau.

Cette découverte est à la base de la présente invention.

Ainsi, conformément à l'un des objets de la présente invention, il est maintenant proposé de nouvelles compositions cosmétiques anhydres, en particulier antisolaires, caractérisées par le fait qu'elles comprennent, dans un support cosmétiquement acceptable constitué d'une phase grasse, au moins un nanopigment d'oxyde de titane et des microsphères creuses déformables d'un copolymère expansé de chlorure de vinylidène et d'acrylonitrile ou de chlorure de vinylidène, d'acrylonitrile et de méthacrylate.

De préférence, ces compositions se présentent sous la forme de crèmes ou de laits ayant une viscosité suffisante pour maintenir en dispersion de manière stable les nanopigments.

Les compositions conformes à l'invention présentent de nombreux avantages.

2

EP 0 755 670 B1

Tout d'abord, le photobleuissement observé lors d'une exposition de ces compositions à la lumière en l'absence d'oxygène est très faible, voire nul.

Ensuite, ces compositions présentent également l'avantage de ne blanchir que très faiblement lors de leur application sur la peau.

Les compositions selon l'invention présentent en outre un facteur de protection solaire élevé, compte tenu du fait que des teneurs très élevées en TiO$_2$ peuvent y être introduites.

Enfin, les compositions conformes à l'invention présentent une stabilité de améliorée, et l'effet gras et collant attaché en général aux produits anhydres est également fortement diminué.

L'invention concerne également l'utilisation de ces compositions comme, ou pour la fabrication de, compositions cosmétiques pour la protection de la peau et/ou des cheveux.

Un autre objet de l'invention est un procédé de traitement cosmétique, en particulier pour la photoprotection de la peau et des cheveux, consistant à appliquer sur ces derniers une quantité efficace d'une composition telle que définie ci-dessus.

Les nanopigments mis en oeuvre dans les compositions selon l'invention sont des nanopigments d'oxyde de titane connus en soi, habituellement utilisés dans le domaine cosmétique, qui peuvent être traités ou non traités. L'oxyde de titane peut se présenter sous forme rutile, anatase ou amorphe, mais de préférence sous forme rutile et/ou anatase.

Par nanopigments, on entend des pigments dont la taille moyenne des particules élémentaires est comprise entre 5 et 100 nm.

Les nanopigments traités peuvent par exemple être traités par l'alumine, la silice, les composés de l'aluminium, les composés du silicium, les composés du sodium, les oxydes de fer, les esters de fer, l'acide stéarique, la glycérine.

Plus particulièrement, les nanopigments traités peuvent être des oxydes de titane traités par :

- la silice et l'alumine tels que les produits « MICROTITANIUM DIOXIDE MT 500 SA » et « MICROTITANIUM DIOXIDE MT 100 SA » de la société TAYCA, et les produits « TIOVEIL Fin », « TIOVEIL OP », « TIOVEIL MOTG » et « TIOVEIL IPM » de la société TIOXIDE,
- l'alumine et le stéarate d'aluminium tels que le produit « MICROTITANIUM DIOXIDE MT 100 T» de la société TAYCA,
- l'alumine et le laurate d'aluminium tels que le produit « MICROTITANIUM DIOXIDE MT 100 S » de la société TAYCA,
- des oxydes de fer et le stéarate de fer tels que le produit « MICROTITANIUM DIOXIDE MT 100 F » de la société TAYCA,
- la silice, l'alumine et la silicone tels que les produits « MICROTITANIUM DIOXIDE MT 100 SAS », « MICROTITANIUM DIOXIDE MT 600 SAS » et « MICROTITANIUM DIOXIDE MT 500 SAS » de la société TAYCA,
- l'hexamétaphosphate de sodium tels que le produit « MICROTITANIUM DIOXIDE MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212 » de la société KEMIRA,
- l'alumine et la silicone tels que le produit « UVT-M262 » de la société KEMIRA.

Les oxydes de titane non traités peuvent par exemple être ceux vendus par la société TAYCA sous les dénominations commerciales « MICROTITANIUM DIOXIDE MT 500 B » ou « MICROTITANIUM DIOXIDE MT 600 B ».

Le ou les nanopigments d'oxyde de titane peuvent être présents dans la composition selon l'invention dans une proportion comprise entre 1 et 30% en poids par rapport au poids total de la composition, de préférence, entre 2 et 25% en poids par rapport au poids total de la composition.

Grâce à l'invention, on peut sans problème introduire des quantités relativement élevées de TiO$_2$, en particulier supérieures à 5% en poids, ou encore supérieures à 10% en poids, ou même supérieures à 15% en poids, et ceci sans être pénalisé par les problèmes de rémanence à l'eau, photobleuissement, et blanchiment évoqués ci-avant.

Une des caractéristiques essentielles des compositions selon l'invention est la présence de particules creuses déformables d'un copolymère expansé de chlorure de vinylidène et d'acrylonitrile ou de chlorure de vinylidène, d'acrylonitrile et de méthacrylate. Par" déformable", il faut comprendre que les particules sont souples et élastiques : après écrasement, elles reprennent leur forme initiale.

Ces particules creuses ont généralement une granulométrie allant de 1 µm à 250 µm. De façon avantageuse, ces particules ont une granulométrie inférieure à 100 µm. En effet, plus les particules sont fines, plus la composition est douce à l'application. De préférence, les particules ont une granulométrie allant de 10 µm à 60 µm.

De façon avantageuse, les particules ont une masse volumique allant de 15 kg/m$^3$ à 200 kg/m$^3$ et mieux supérieure à 40 kg/m$^3$ et/ou inférieure à 100 kg/m$^3$ et notamment allant de 60 kg/m$^3$ à 80 kg/m$^3$.

On peut, par exemple, utiliser un copolymère contenant : de 1 % à 60 % de motifs dérivés du chlorure de vinylidène, de 20 % à 90 % de motifs dérivés d'acrylonitrile et de 1 % à 50 % de motifs dérivés d'un monomère acrylique, la

3

somme des pourcentages (en poids) étant égale à 100. Le monomère acrylique est par exemple un acrylate ou méthacrylate de méthyle et, en particulier le méthacrylate. Ces particules se présentent notamment à l'état sec ou hydraté.

Ce copolymère est non toxique et non irritant pour la peau.

De préférence, les particules se présentent sous forme de microsphères.

Les particules de l'invention peuvent être obtenues, par exemple, selon les procédés des brevets et demandes de brevet EP-56 219, EP-348 372, EP-486 080, EP-320 473, EP-112 807 et US-3 615 972.

La cavité interne des particules contient en principe un gaz qui peut être de l'air, de l'azote ou un hydrocarbure comme l'isobutane ou l'isopentane.

Les particules creuses utilisables dans l'invention sont en particulier celles vendues sous la marque Expancel par la Société Nobel Casco telles que l'Expancel 551 DE 20 de granulométrie de 30 µm et de masse volumique de 65 kg/m$^3$ environ ; l'Expancel 551 DE 50 de granulométrie de 40 µm.

Dans les compositions de l'invention, on utilise de préférence de 0,2 % à 3 % en poids de particules et mieux de 0,5 % à 1,5 % en poids par rapport au poids total de la composition.

Dans les compositions selon l'invention, la phase grasse contient des composés classiquement utilisés dans le domaine de la fabrication des compositions cosmétiques anhydres tels que notamment des huiles, des composés cireux, des émulsionnants, des gélifiants.

Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline), végétales (huile d'amande douce, de macadamia, de pépin de cassis), synthétiques comme le perhydrosqualène; les alcools, les acides ou les esters gras (palmitate d'octyle, lanolate d'isopropyle, les triglycérides dont ceux des acides caprique/caprylique); les esters et éthers gras oxyéthylénés ou oxypropylénés; les huiles siliconées (cyclométhicone, polydiméthysiloxanes ou PDMS) ou fluorées; les polyalkylènes. De préférence, les huiles sont des polyalkylènes tels que les polybutènes ou par exemple le polydécène hydrogéné vendu sous la dénomination commerciale « Silkflo S 366 NF » par Albemarle. On peut également utiliser le palmitate d'isopropyle vendu sous la dénomination commerciale « Estol 1517 » par Unichema.

Comme composés cireux, on peut citer l'huile de jojoba, la paraffine, la cire de carnauba, la cire d'abeille, l'huile de ricin hydrogénée. De préférence, la cire utilisée est l'huile de ricin hydrogénée vendue sous la dénomination commerciale « Cutina HR » par Henkel.

Comme émulsionnant, on peut utiliser les esters d'acides gras et de polyéthylène glycol (PEG), les esters d'acides gras et de glycérol (stéarate de glycéryle) ou les esters d'acides gras et de sucre (stéarate de sorbitane), ainsi que leurs dérivés polyoxyéthylénés ou polyoxypropylénés, les cyclométhicones et diméthicones copolyols, les tensioactifs anioniques (alkylphosphate de K ou de Na), les alcools gras polyalcoxylés.

De préférence, on utilise les alcools gras polyalcoxylés tels que les alcools butyliques oxypropylénés, les alcools capryliques oxyéthylénés, les alcools cétyliques oxyéthylénés.

Un émulsionnant convenant particulièrement bien pour les compositions selon l'invention est l'alcool butylique oxypropyléné 14 OP (oxypropylène) vendu sous la dénomination commerciale « Ucon Fluid AP » par Amerchol.

Comme gélifiants, on peut citer les argiles modifiées (bentones), les sels métalliques d'acides gras (stéarate d'aluminium), les copolymères éthylène/acrylate, les silices, les polyéthylènes, les silicates de calcium ou encore l'éthylcellulose.

De préférence, la phase grasse a une viscosité suffisante pour maintenir en dispersion de manière stable les nanopigments.

Les compositions de l'invention peuvent éventuellement contenir des filtres organiques, ainsi que d'autres types de pigments minéraux.

Bien entendu, l'homme de l'art veillera à choisir le ou les éventuels composés complémentaires évoqués ci-avant et/ou leurs quantités de manière telle que les propriétés avantageuses, et notamment de réduction des phénomènes de photobleuissement et de blanchiment, attachées intrinsèquement à la composition conforme à l'invention ne soient pas, ou substantiellement pas, altérées par la ou les adjonctions envisagées.

De préférence, les compositions selon l'invention se présentent sous la forme d'une crème anhydre.

Les compositions selon l'invention présentent généralement un facteur de protection solaire d'au moins 4, de préférence d'au moins 10, et encore plus préférentiellement d'au moins 20, le facteur de protection solaire (SPF) s'exprimant mathématiquement par le rapport du temps d'irradiation nécessaire pour atteindre le seuil érythématogène avec le filtres UV au temps nécessaire pour atteindre le seuil érythématogène sans filtres UV.

Des exemples concrets illustrant l'invention, vont maintenant être donnés.

## EXEMPLE 1 :

On a réalisé des tests comparatifs afin de mettre en évidence l'amélioration apportée par l'utilisation de microsphères creuses déformables d'un copolymère expansé de chlorure de vinylidène et d'acrylonitrile ou de chlorure de vinylidène, d'acrylonitrile et de méthacrylate, dans une composition solaire anhydre contenant du TiO$_2$.

Pour cela, on a réalisé une crème anhydre contenant un certain pourcentage x d'Expancel et 20% d'oxyde de titane, dans un support composé d'un alcool gras polyalcoxylé (alcool butylique oxypropyléné), d'un corps gras de la classe des esters (palmitate d'isopropyle), d'une cire dont le point de fusion est compris entre 60 et 80°C (huile de ricin hydrogénée), d'un corps gras épaississant (monostéarate de glycérol) et d'une huile (polydécène hydrogéné).

On a ainsi réalisé cinq crèmes différentes A, B, C, D et E en faisant varier la teneur x de 0 à 2%.

Pour ces cinq crèmes, le photobleuissement a été évalué selon le protocole suivant : les compositions ont été introduites dans des boîtes en plastique transparentes aux UV ( boîtes polystyrène cristal 50x40x6 cm$^3$) et exposées aux UV (SUNTEST CPS Heraeus) pendant 1H. Les mesures colorimétriques ont été effectuées à l'aide d'un colorimètre Minolta CM1000 : une première mesure a été relevée juste avant l'exposition aux UV (T0) et une deuxième après une heure d'exposition aux UV (T1H).

Les résultats sont exprimés dans le système (L, a, b) dans lequel L représente la luminance, a représente l'axe rouge-vert (-a = vert, +a = rouge) et b représente l'axe jaune-bleu (-b =bleu, +b =jaune).

Pour l'évaluation du photobleuissement, on s'intéresse à $\Delta b$, qui mesure la variation de la couleur bleue, à $\Delta L$, qui mesure le noircissement de la composition et à $\Delta E$, qui mesure la variation de couleur totale. Plus précisément, $\Delta b$ et $\Delta L$ sont définis par $\Delta b = b_{T1H} - b_{T0}$ et $\Delta L = L_{T1H} - L_{T0}$. Plus $\Delta b$ est faible, plus la protection contre le photobleuissement est efficace.

$\Delta E$ est calculé à partir des variations $\Delta L$, $\Delta a$ et $\Delta b$ selon la formule suivante :

$$\Delta E = \sqrt{(\Delta L)^2 + (\Delta a)^2 + (\Delta b)^2}$$

Pour l'évaluation du blanchiment, des tests ont été réalisés sur cinq modèles. L'intensité du blanchiment (lb) est mesurée selon une échelle graduée de 0 à 5. La valeur 0 correspond à une composition très blanchissante tandis que la valeur 5 correspond à une composition très peu blanchissante.

Les résultats sont consignés dans le tableau (I) suivant :

Tableau (I)

| Formule | x% | $\Delta L$ | $\Delta b$ | $\Delta E$ | lb |
|---|---|---|---|---|---|
| A (comparatif) | 0 | 10,7 | 7,7 | 13,60 | 2 |
| B | 0,2 | 8,0 | 4,45 | 9,55 | 2,9 |
| C | 0,5 | 3,15 | 0,27 | 3,30 | 2,8 |
| D | 1 | 4,6 | 0,95 | 4,80 | 3,6 |
| E | 2 | 2,75 | 0,14 | 2,80 | 3,7 |

Ces résultats montrent clairement que l'introduction de microsphères creuses déformables d'un copolymère expansé de chlorure de vinylidène et d'acrylonitrile ou de chlorure de vinylidène, d'acrylonitrile et de méthacrylate au sein d'une crème anhydre comportant un oxyde de titane diminue significativement le photobleuissement dû à la présence dudit oxyde de titane ainsi que le blanchiment de la composition lors de l'application de cette dernière sur la peau.

Par ailleurs, les compositions B à E, conformes à l'invention, se sont révélées très stables.

**EXEMPLE 2** :

La composition suivante donne un exemple concret d'une crème solaire anhydre haute protection conforme à l'invention. Les quantités sont exprimées en % de poids par rapport au poids total de la composition :

Phase A :

| | |
|---|---|
| - alcool butylique oxypropyléné 14 OP vendu sous la dénomination commerciale « Ucon Fluid AP » par Amerchol (émulsionnant) | 12% |
| - palmitate d'isopropyle vendu sous la dénomination commerciale « Estol 1517 » par Unichema | 12% |
| - huile de ricin hydrogénée vendue sous la dénomination commerciale « Cutina HR » par Henkel | 7,5% |
| - monostéarate de glycérol vendu sous la dénomination commerciale « Tegin 90 » par Goldschmidt | 0,25% |

Phase B :

| | |
|---|---|
| - oxyde de titane vendu sous la dénomination commerciale « MT 100T » par Tayca | 20% |
| - silicate de magnésium vendu sous la dénomination commerciale « Talc Luzenac 15 M00 » par Luzenac | 5% |
| - microsphères expansées de copolymère chlorure de vinylidène / acrylonitrile / méthacrylate de méthyle contenant de l'isobutane vendu sous la dénomination commerciale « Expancel 551 DE » par Nobel Casto | 0,5% |
| - polydécène hydrogéné (PM 549) vendu sous la dénomination commerciale « Silkflo S 366 NF » par Albemarle | qsp 100% |

Cette crème a été réalisée de la façon suivante : on a chauffé la phase A à 90°C afin de l'homogénéiser. Puis on a préparé la pâte pigmentaire en mélangeant l'oxyde de titane au polydécène hydrogéné. On a ajouté à ce mélange le talc puis l'Expancel. La phase B ainsi réalisée a été chauffée à 90°C. Puis on a mélangé les deux phases A et B au Rayneri. Une fois le mélange pris en masse, on a de nouveau chauffé à 90°C. Puis on a turbiné le mélange au Turrax pendant 10 minutes. On a ensuite laissé refroidir la crème à température ambiante sous agitation Rayneri.

Cette crème présente un facteur de protection solaire de 30, est très faiblement sujette au photobleuissement et ne blanchit pratiquement pas lorsqu'on l'applique sur la peau.

Elle est par ailleurs très stable.

**Revendications**

**1.** Composition cosmétique anhydre, caractérisée par le fait qu'elle comprend, dans un support cosmétiquement acceptable constitué d'une phase grasse, au moins un nanopigment d'oxyde de titane et des microsphères creuses déformables d'un copolymère expansé de chlorure de vinylidène et d'acrylonitrile ou de chlorure de vinylidène, d'acrylonitrile et de méthacrylate.

**2.** Composition selon la revendication 1, caractérisée en ce que les microsphères ont une granulométrie allant de 1 μm à 250 μm.

**3.** Composition selon la revendication 2, caractérisée en ce que les microsphères ont une granulométrie inférieure à 100 μm.

**4.** Composition selon la revendication 3, caractérisée en ce que les microsphères ont une granulométrie allant de 10 μm à 60 μm.

**5.** Composition selon l'une quelconque des revendications précédentes, caractérisée en ce que les microsphères ont une masse volumique allant de 15 kg/m$^3$ à 200 kg/m$^3$.

6. Composition selon revendication 5, caractérisée en ce que les microsphères ont une masse volumique allant de 40 kg/m$^3$ à 100 kg/m$^3$.

7. Composition selon l'une quelconque des revendications précédentes, caractérisée en ce que le nanopigment d'oxyde de titane est choisi parmi les oxydes de titane traités par l'alumine, la silice, les composés de l'aluminium, les composés du silicium, les composés du sodium, les oxydes de fer, les esters de fer, l'acide stéarique, la glycérine.

8. Composition selon l'une quelconque des revendications précédentes, caractérisée en ce que les microsphères représentent de 0,1 % à 3 % du poids total de la composition.

9. Composition selon la revendication 8, caractérisée en ce que les microsphères représentent de 0,5 % à 1,5 % du poids total de la composition.

10. Composition selon l'une quelconque des revendications précédentes, caractérisée par le fait que le ou les nanopigments d'oxyde de titane sont présents dans la composition dans une proportion comprise entre 1 et 30% en poids par rapport au poids total de la composition.

11. Composition selon la revendication 10, caractérisée par le fait que le ou les nanopigments d'oxyde de titane sont présents dans la composition dans une proportion comprise entre 2 et 25% en poids par rapport au poids total de la composition.

12. Composition selon la revendication 11, caractérisée par le fait que le ou les nanopigments d'oxyde de titane sont présents dans la composition dans une proportion d'au moins 5% en poids par rapport au poids total de la composition.

13. Composition selon la revendication 12, caractérisée par le fait que le ou les nanopigments d'oxyde de titane sont présents dans la composition dans une proportion d'au moins 10% en poids par rapport au poids total de la composition.

14. Composition selon la revendication 13, caractérisée par le fait que le ou les nanopigments d'oxyde de titane sont présents dans la composition dans une proportion d'au moins 15% en poids par rapport au poids total de la composition.

15. Composition selon l'une quelconque des revendications précédentes, caractérisée par le fait que ladite phase grasse a une viscosité suffisante pour maintenir en dispersion de manière stable lesdits nanopigments.

16. Composition selon l'une quelconque des revendications précédentes, caractérisée par le fait que ladite phase grasse comprend au moins une huile choisie parmi les huiles minérales (vaseline), végétales (huile d'amande douce, de macadamia, de pépin de cassis), synthétiques comme le perhydrosqualène; les alcools, les acides ou les esters gras (palmitate d'octyle, lanolate d'isopropyle, les triglycérides dont ceux des acides caprique/caprylique); les esters et éthers gras oxyéthylénés ou oxypropylénés; les huiles siliconées (cyclométhicone, polydiméthysiloxanes ou PDMS) ou fluorées; les polyalkylènes.

17. Composition selon l'une quelconque des revendications précédentes, caractérisée par le fait que ladite phase grasse comprend au moins une cire choisie parmi l'huile de jojoba, la paraffine, la cire de carnauba, la cire d'abeille, l'huile de ricin hydrogénée.

18. Utilisation des compositions définies à l'une quelconque des revendications 1 à 17 comme, ou pour la fabrication de, compositions cosmétiques pour la protection de la peau et/ou des cheveux.

19. Procédé de traitement cosmétique, en particulier pour la photoprotection de la peau ou des cheveux consistant à appliquer sur ces derniers une quantité efficace d'une composition définie à l'une des revendications 1 à 17.

## Claims

1. Anhydrous cosmetic composition, characterized in that it comprises, in a cosmetically acceptable vehicle consisting of a fatty phase, at least one titanium oxide nanopigment and deformable hollow microspheres of an expanded copolymer of vinylidene chloride and acrylonitrile or of vinylidene chloride, acrylonitrile and methacrylate.

2. Composition according to Claim 1, characterized in that the microspheres have a particle size ranging from 1 μm to 250 μm.

3. Composition according to Claim 2, characterized in that the microspheres have a particle size of less than 100 μm.

4. Composition according to Claim 3, characterized in that the microspheres have a particle size ranging from 10 μm to 60 μm.

5. Composition according to any one of the preceding claims, characterized in that the microspheres have a density ranging from 15 kg/m$^3$ to 200 kg/m$^3$.

6. Composition according to Claim 5, characterized in that the microspheres have a density ranging from 40 kg/m$^3$ to 100 kg/m$^3$.

7. Composition according to any one of the preceding claims, characterized in that the titanium oxide nanopigment is chosen from titanium oxides treated with alumina, silica, aluminium compounds, silicon compounds, sodium compounds, iron oxides, iron esters, stearic acid and glycerol.

8. Composition according to any one of the preceding claims, characterized in that the microspheres represent from 0.1% to 3% of the total weight of the composition.

9. Composition according to Claim 8, characterized in that the microspheres represent from 0.5% to 1.5% of the total weight of the composition.

10. Composition according to any one of the preceding claims, characterized in that the titanium oxide nanopigment(s) is(are) present in the composition in a proportion of between 1 and 30% by weight relative to the total weight of the composition.

11. Composition according to Claim 10, characterized in that the titanium oxide nanopigment(s) is (are) present in the composition in a proportion of between 2 and 25% by weight relative to the total weight of the composition.

12. Composition according to Claim 11, characterized in that the titanium oxide nanopigment(s) is (are) present in the composition in a proportion of at least 5% by weight relative to the total weight of the composition.

13. Composition according to Claim 12, characterized in that the titanium oxide nanopigment (s) is (are) present in the composition in a proportion of at least 10% by weight relative to the total weight of the composition.

14. Composition according to Claim 13, characterized in that the titanium oxide nanopigment(s) is (are) present in the composition in a proportion of at least 15% by weight relative to the total weight of the composition.

15. Composition according to any one of the preceding claims, characterized in that the said fatty phase has a viscosity which is sufficient to maintain the said nanopigments stably in dispersion.

16. Composition according to any one of the preceding claims, characterized in that the said fatty phase comprises at least one oil chosen from mineral oils (petrolatum), plant oils (sweet almond oil, macadamia oil, blackcurrant seed oil) and synthetic oils such as perhydrosqualene; fatty alcohols, acids or esters (octyl palmitate, isopropyl lanolate, triglycerides including capric/caprylic acid triglycerides); oxyethylenated or oxypropylenated fatty esters and ethers; silicone-containing oils (cyclomethicone, polydimethylsiloxanes or PDMS) or fluoro oils; polyalkylenes.

17. Composition according to any one of the preceding claims, characterized in that the said fatty phase comprises at least one wax chosen from jojoba oil, paraffin, carnauba wax, beeswax and hydrogenated castor oil.

18. Use of the compositions defined in any one of Claims 1 to 17 as, or for the manufacture of, cosmetic compositions for protecting the skin and/or the hair.

19. Cosmetic treatment process, in particular for the photoprotection of the skin or the hair, which consists in applying an effective amount of a composition defined in one of Claims 1 to 17 to the skin or the hair.

**Patentansprüche**

1. Wasserfreie kosmetische Zusammensetzung, dadurch gekennzeichnet, daß sie in einem kosmetisch akzeptablen Träger, der aus einer Fettphase besteht, mindestens ein Titandioxid-Nanopigment und deformierbare hohle Mikrokügelchen aus einem expandierten Vinylidenchlorid/Acrylnitril- oder Vinylidenchlorid/Acrylnitril/Methacrylat-Copolymer enthält.

2. Zusammensetzung nach Anspruch 1, dadurch gekennzeichnet, daß die Mikrokügelchen eine Partikelgröße von 1 bis 250 µm aufweisen.

3. Zusammensetzung nach Anspruch 2, dadurch gekennzeichnet, daß die Mikrokügelchen eine Partikelgröße unter 100 µm aufweisen.

4. Zusammensetzung nach Anspruch 3, dadurch gekennzeichnet, daß die Mikrokügelchen eine Partikelgröße von 10 bis 60 µm aufweisen.

5. Zusammensetzung nach einem der vorhergehenden Ansprüche, dadurch gekennzeichnet, daß die Mikrokügelchen eine Dichte von 15 bis 200 kg/m$^3$ aufweisen.

6. Zusammensetzung nach Anspruch 5, dadurch gekennzeichnet, daß die Mikrokügelchen eine Dichte von 40 bis 100 kg/m$^3$ aufweisen.

7. Zusammensetzung nach einem der vorhergehenden Ansprüche, dadurch gekennzeichnet, daß das Titandioxid-Nanopigment unter Titandioxiden ausgewählt ist, die mit Aluminiumoxid, Siliciumdioxid, Aluminiumverbindungen, Siliciumverbindungen, Natriumverbindungen, Eisenoxiden, Eisenestern, Stearinsäure, Glycerin behandelt sind.

8. Zusammensetzung nach einem der vorhergehenden Ansprüche, dadurch gekennzeichnet, daß die Mikrokügelchen 0,1 bis 3 % des Gesamtgewichts der Zusammensetzung ausmachen.

9. Zusammensetzung nach Anspruch, dadurch gekennzeichnet, daß die Mikrokügelchen 0,5 bis 1,5 % des Gesamtgewichts der Zusammensetzung ausmachen.

10. Zusammensetzung nach einem der vorhergehenden Ansprüche, dadurch gekennzeichnet, daß das oder die Titandioxid-Nanopigmente in der Zusammensetzung in einem Anteil von 1 bis 30 Gew.-%, bezogen auf das Gesamtgewicht der Zusammensetzung, enthalten sind.

11. Zusammensetzung nach Anspruch 10, dadurch gekennzeichnet, daß das oder die Titandioxid-Nanopigmente in der Zusammensetzung in einem Anteil von 2 bis 25 Gew.-%, bezogen auf das Gesamtgewicht der Zusammensetzung, enthalten sind.

12. Zusammensetzung nach Anspruch 11, dadurch gekennzeichnet, daß das oder die Titandioxid-Nanopigmente in der Zusammensetzung in einem Anteil von mindestens 5 Gew.-%, bezogen auf das Gesamtgewicht der Zusammensetzung, enthalten sind.

13. Zusammensetzung nach Anspruch 12, dadurch gekennzeichnet, daß das oder die Titandioxid-Nanopigmente in der Zusammensetzung in einem Anteil von mindestens 10 Gew.-%, bezogen auf das Gesamtgewicht der Zusammensetzung, enthalten sind.

14. Zusammensetzung nach Anspruch 13, dadurch gekennzeichnet, daß das oder die Titandioxid-Nanopigmente in der Zusammensetzung in einem Anteil von mindestens 15 Gew.-%, bezogen auf das Gesamtgewicht der Zusammensetzung, enthalten sind.

15. Zusammensetzung nach einem der vorhergehenden Ansprüche, dadurch gekennzeichnet, daß die Fettphase eine Viskosität aufweist, die so groß ist, daß die obengenannten Nanopigmente in stabiler Weise dispergiert bleiben.

16. Zusammensetzung nach einem der vorhergehenden Ansprüche, dadurch gekennzeichnet, daß die Fettphase mindestens ein Öl enthält, das unter Mineralölen (Vaseline), pflanzlichen Ölen (Süßmandelöl, Macadamiaöl, Johannisbeerkernöl), synthetischen Ölen wie Perhydrosqualen; Fettalkoholen, Fettsäuren und Fettestern (Octylpalmitat, Isopropyllanolat, Triglyceride), darunter die Triglyceride von Caprin-/Caprylsäure); ethoxylierten und propoxylierten Fettestern und Fettethern; Siliconölen (Cyclomethicon, Polydimethylsiloxanen und PDMS), fluorierten Ölen; Polyalkylenen ausgewählt ist.

17. Zusammensetzung nach einem der vorhergehenden Ansprüche, dadurch gekennzeichnet, daß die obengenannte Fettphase mindestens ein Wachs enthält, das unter Jojobaöl, Paraffin, Carnaubawachs, Bienenwachs, hydriertem Ricinusöl ausgewählt ist.

18. Verwendung der in einem der Ansprüche 1 bis 17 definierten Zusammensetzungen als kosmetische Zusammensetzungen oder für die Herstellung kosmetischer Zusammensetzungen für den Schutz der Haut und/oder der Haare.

19. Verfahren zur kosmetischen Behandlung, insbesondere für den Lichtschutz der Haut oder der Haare, das darin besteht, auf die Haut oder die Haare eine wirksame Menge einer in einem der Ansprüche 1 bis 17 definierten Zusammensetzungen aufgetragen wird.